Europäisches Patentamt

European Patent Office

Office européen des brevets

(11) Veröffentlichungsnummer: **0 180 735**
**B1**

(12) **EUROPÄISCHE PATENTSCHRIFT**

(45) Veröffentlichungstag der Patentschrift: **12.12.90**

(21) Anmeldenummer: **85111103.9**

(22) Anmeldetag: **03.09.85**

(51) Int. Cl.⁵: **A 61 B 5/00**, A 61 B 5/04

(54) Elektrodenanordung für Messwertaufnehmer.

(30) Priorität: **06.11.84 DE 3440401**

(43) Veröffentlichungstag der Anmeldung:
**14.05.86 Patentblatt 86/20**

(45) Bekanntmachung des Hinweises auf die
Patenterteilung:
**12.12.90 Patentblatt 90/50**

(84) Benannte Vertragsstaaten:
**CH DE GB LI**

(56) Entgegenhaltungen:
**EP-A-0 077 054**
**DE-A-1 466 768**
**DE-A-2 930 663**
**US-A-4 393 584**

(73) Patentinhaber: **Drägerwerk Aktiengesellschaft
Moislinger Allee 53-55
D-2400 Lübeck 1 (DE)**

(72) Erfinder: **Hölscher, Uvo, Dr.-Ing.
Stettiner Strasse 6a
D-2406 Stockelsdorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Elektrodenanordnung für Meßwertaufnehmer zur kombinierten Messung verschiedener physiologischer Größen nach dem Oberbegriff des Patentanspruchs.

Eine solche Elektrodenanordnung für einen Meßwertaufnehmer ist aus der EP-A-0,077,054 bekannt. Bei diesem Meßwertaufnehmer ist an einem Meßkopfkörper neben der Elektrodenanordnung zur Aufnahme einer ersten physiologischen Meßgröße eine weitere Elektrodenanordnung vorgesehen, welche eine erste Elektrode auf der Kontaktfläche zugewandten Seite des Meßkopfkörpers und eine zweite Elektrode auf der Rückseite des Meßkopfkörpers aufweist. Mit einen solchen Meßwertaufnehmer werden beispielsweise über die erste Elektrodenanordnung der transcutane Sauerstoffpartialdruck und mit der weiteren Elektrodenanordnung eine zweite physiologische Größe, wie z.B. die Herzaktionsspannung (EKG) gemessen. Dabei dient die erste Elektrode auf der Kontaktfläche zugewandten Seite des Meßkopfkörpers als Meßelektrode und die zweite Elektrode auf der Rückseite des Meßkopfkörpers als Gegenelektrode.

Bei der bekannten Elektrodenanordnung wird das Klebemittel auf die dafür vorgesehene Auflagefläche des Meßkopfkörpers aufgetragen, wobei die Meßelektrode von diesem Klebemittel umgeben wird. Eine solche Klebung soll die elektrische Isolierung der Meßelektrode gegenüber der Gegenelektrode herstellen. Dabei ist jedoch nur ein geringer Außenbereich der gesamten Auflagefläche für die Klebehaftung verfügbar, so daß ihre Haltbarkeit begrenzt ist. Diese wird noch dadurch vermindert, daß von außen in die Klebeschicht hinein beispielsweise Körperflüssigkeiten eindringen können und so die Haftfähigkeit der Klebeschicht herabsetzen.

Bei der Anordnung von Klebeflächen neben den mit der Haut zu verbindenden Elektrodenflächen sind zwei sich anscheinend widersprechende Forderungen zu erfüllen: die Flächen der Elektroden sollen zum einen möglichst groß sein, damit sie eine möglichst geringe Impedanz zum Körper hin aufweisen und somit störunempfindlich sind, und außerdem sollen die Klebeflächen der Meßwertaufnehmer möglichst groß sein, damit sie sich in ihrer praktischen Anwendung auch unter größerer Zug- und Querkraftbelastung nicht von der Kontaktfläche fortbewegen können; zum anderen soll die Gesamtfläche aus Klebefläche und Elektrodenfläche möglichst klein sein, damit die Sensoren auch an kleinflächigen Körperteilen des Menschen, sogar am kindlichen Kopf des Ungeborenen, anbringbar sind. Im allgemeinen werden Elektroden zur Aufnahme von physiologischen Größen mit einem ionenleitenden Gel zur Kontaktierung mit der Haut versehen. Dessen Haftkraft ist jedoch für größere Lasten nicht ausreichend, so daß ein Einsatz bei den vergleichsweise voluminösen und schweren Meßwertaufnehmern zur transcutanen Sauerstoffmessung keine zuverlässigen Haftbedingungen auf der Kontaktfläche unter den in der Praxis auftretenden, erschwerten Bedingungen gewährleistet.

Die Haftkraft wird bei einem Meßwertaufnehmer, der zur Messung physiologischer Größen geeignet ist, weiter verringert, da eine Aussparung für die entsprechend notwendigen Elektrodenanordnungen in der Klebefläche notwendig ist.

In der US-A-4,393,584 ist eine Meßelektrode zur Aufnahme eines Herzaktionspotentials angegeben, die mittels eines elektrisch leitenden Haftklebers auf die Haut der zu untersuchenden Person gebracht werden kann.

Die Aufgabe vorliegender Erfindung wird daher darin gesehen, die Kontaktklebefläche eines Meßwertaufnehmers zur kombinierten Messung von mehreren physiologischen Größen so auszubilden und eine entsprechende Befestigungsart auf der Haut zu finden, daß die Elektrode zur Aufnahme der zweiten physiologischen Meßgröße und die Klebefläche zur Befestigung des Meßwertaufnehmers sich gegenseitig nicht behindern und eine in der Praxis tolerable Gesamtgröße besitzen.

Diese Aufgabe wird bei einer Elektrodenanordnung der genannten Art durch die kennzeichnenden Merkmale des Patentanspruchs gelöst.

Bei der erfindungsgemäßen Ausbildung der Klebeschicht ist gewährleistet, daß die Elektrode zur Messung einer bioelektrischen Größe, wie zum Beispiel des Elektrokardiogramms, von ihrer Gegenelektrode durch einen isolierenden Bereich der Klebefläche getrennt ist, so daß weder Körperflüssigkeiten, die bei der Anwendung des Meßwertaufnehmers beim Feten unter der Geburt in Form von Fruchtwasser auftreten könnten, oder andere von außen herantretende Flüssigkeiten weder einen Kurzschluß zwischen Meß- und Gegenelektrode noch eine Verminderung der Potentialdifferenz zwischen beiden Elektroden bewirken können. Die großflächige Anklebung des Meßwertaufnehmers sorgt für eine gesicherte Halterung auch unter erhöhten Zugspannungen und Querkräften entlang der Klebefläche. Der Bereich der Klebefläche aus elektrisch isolierendem Material erhöht in vorteilhafter Weise die elektrische Isolation der leitenden Dotierungszone gegenüber ihrer Umgebung.

Durch die Anbringung von Meß- und Gegenelektrode auf einem gemeinsamen Ringkörper um den Meßkopf wird es ermöglicht, den Ringkörper mit seiner Meß- und Gegenelektrode im Bedarfsfalle lediglich zur Messung einer physiologischen Größe, wie z.B. die Herzaktionsspannung, einzusetzen. Dazu wird der Meßkopf für die transcutane Messung durch einen passenden Blindstopfen ersetzt. Umgekehrt kann an einem vorhandenen Meßkopf, eine vollständige in sich funktionsfähige Zusatzelektrodenanordnung, beispielsweise zur Bestimmung eines EKG, angesetzt und als eine Einheit betrieben werden.

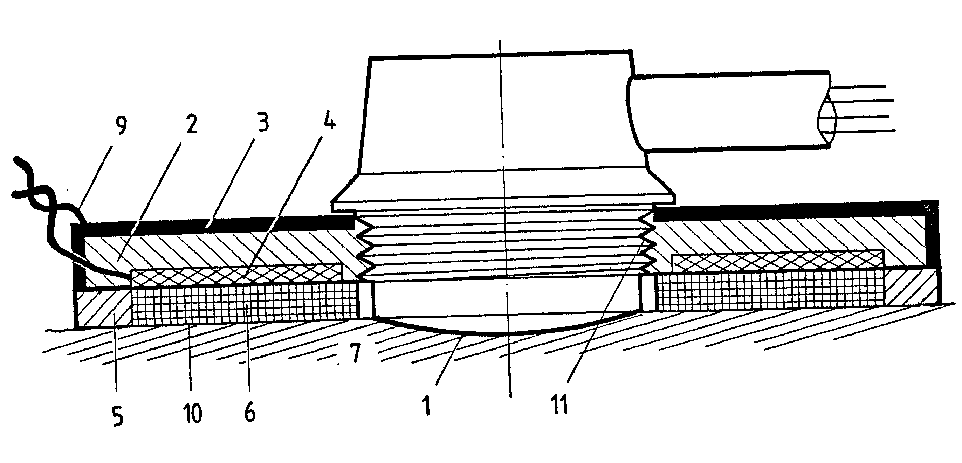

Anhand der in der Zeichnung angegebenen Figur ist ein Ausführungsbeispiel der Erfindung näher erläutert.

Der dargestellte Meßwertaufnehmer zeigt einen Meßkopf 1 zur Messung einer ersten physiologischen Größe, zum Beispiel des transcutanen Sauerstoffpartialdruckes. Ihn umgibt ein Ringkörper 2, der mit einem Gewindeteil 11 in seiner zentrischen Aussparung den Meßkopf 1 aufnimmt. An dem Ringkörper 2 ist eine Elektrodenanordnung zur Messung einer zweiten physiologischen Größe, wie zum Beispiel der Herzaktionsspannung, angebracht. Diese enthält eine Meßelektrode 4, welche in eine ringförmige Ausnehmung des Ringkörpers 2 eingelassen ist. Auf der Meßelektrode 4 abgewandten Seite des Ringkörpers 2 ist eine Gegenelektrode 3 angebracht. Beide-Elektroden 3, 4 sind über Anschlußleitungen 9 mit einer nicht dargestellten Auswerteeinheit verbunden. Der Ringkörper 2 mit dem darin enthaltenen Meßkopf 1 ist über eine Klebefläche 10 an der Haut 7 zur Aufnahme der Meßgrößen befestigt. Die Klebefläche 10 wird durch ein klebendes, elektrisch isolierendes Randstück begrenzt. Zwischen Randstück 5 und Meßkopf 1 schließt sich eine Dotierungszone 6 an, welche einen bekannten elektrolytisch oder elektrisch leitenden Haftkleber enthält; dieser stellt die elektrische Verbindung zwischen Haut 7 und Meßelektrode 4 her.

## Patentanspruch

Elektrodenanordnung für Meßwertaufnehmer zur kombinierten Messung verschiedener physiologischer Größen mit einem Meßkopf (1) zur Aufnahme einer ersten physiologischen Größe und Mitteln zur Messung einer zweiten physiologischen Größe, welche aus einer ersten, auf einer dem Meßkopf (1) zugewandten Seite eines elektrisch isolierenden Ringkörpers (2) befindlichen Meßelektrode (4) sowie einer zweiten, dem Meßkopf (1) abgewandten Gegenelektrode (3) bestehen, wobei die Meßelektrode (4) mittels eines elektrisch leitfähigen Haftklebers (6) auf die zu untersuchende Haut (7) befestigbar ist, dadurch gekennzeichnet, daß der Ringkörper (2) auf der Meßelektrode (4) abgewandten Seite die Gegenelektrode (3) trägt, daß die Meßelektrode (4) in einer von einem elektrisch isolierenden Randstück (5) begrenzten und zur Haut (7) hin geöffneten Ausnehmung im Ringkörper (2) aufgenommen ist, und daß die Ausnehmung durch den Haftkleber (6) ausgefüllt ist.

## Revendication

Agencement d'électrodes pour capteurs de valeurs mesurées, destiné à la mesure combinée de différentes grandeurs physiologiques, comportant une tête de mesure (1) pour la détection d'une première grandeur physiologique et des moyens pour mesurer une deuxième grandeur physiologique, lesquels sont constitués d'une première électrode de mesure (4), se trouvant sur un côté tourné vers la tête de mesure (1), d'un corps annulaire (2) isolant électriquement, ainsi que d'une deuxième contre-électrode (3), opposée à la tête de mesure (1), l'électrode de mesure (4) pouvant être fixée sur la peau (7) à analyser, au moyen d'un adhésif (6) électriquement conducteur, caractérisé en ce que le corps annulaire (2) porte la contre-électrode (3) sur le côté opposé à l'électrode de mesure (4), en ce que l'électrode de mesure (4) est logée dans un évidement du corps annulaire (2), limité par une pièce de bordure (5) isolante électriquement et ouvert vers la peau (7) et en ce que l'évidement est rempli par l'adhésif (6).

## Claim

An electrode arrangement for a measuring transducer for combined measurement of various physiological quantities with a measuring head (1) for receiving a first physiological quantity and means for measuring a second physiological quantity, which consist of a first measuring electrode (4), located on a side facing the measuring head (1) of an electrically insulating annular body (2) as well as a second counter electrode (3) facing away from the measuring head (1), wherein the measuring electrode (4) is able to be secured by means of an electrically conductive adhesive (6) to the skin (7) to be examined, characterised in that the annular body (2) carries the counter electrode (3) on the side facing away from the measuring electrode (4), in that the measuring electrode (4) is received in a recess in the annular body (2) open to the skin (7) and defined by an electrically insulating edge piece (5), and in that the recess is filled by the adhesive (6).

EP 0 180 735 B1